# FASCICULE DE BREVET EUROPEEN

(11) **EP 3 890 667 B1**
(45) Date de publication et mention de la délivrance du brevet: **19.02.2025**
(21) Numéro de dépôt: 19813026.2
(22) Date de dépôt: 05.12.2019
(51) Int. Cl.: A61F 9/007, A61B 34/30, A61B 90/25, B25J 9/16, A61F 9/008, A61B 90/20, A61B 34/20, A61B 3/10, A61B 3/13, A61B 90/00

(54) **DISPOSITIF MÉDICAL POUR LA CHIRURGIE DE L'OEIL**
MEDIZINPRODUKT FÜR AUGENOPERATION
MEDICAL DEVICE FOR EYE SURGERY

(30) Priorité: 06.12.2018 FR 1872462
(43) Date de publication de la demande: 13.10.2021
(73) Titulaire: Université de Montpellier, 34090 Montpellier (FR); Centre National de la Recherche Scientifique (CNRS), 75016 Paris (FR); Acusurgical, 34000 Montpellier (FR)
(72) Inventeur: SPUHLER, Christoph, 34000 Montpellier (FR); POIGNET, Philippe, 34150 Gignac (FR); HADDAB, Yassine, 34090 Montpellier (FR)
(74) Mandataire: Gevers & Orès
(86) Numéro de dépôt international: PCT/EP2019/083900
(87) Numéro de publication internationale: WO 2020/115249

(56) Documents cités:
- WO-A1-2017/162981
- US-A1- 2017 209 042
- MINGCHUAN ZHOU ET AL: "Towards Robotic Eye Surgery: Marker-free, Online Hand-eye Calibration using Optical Coherence Tomography Images", ARXIV.ORG, CORNELL UNIVERSITY LIBRARY, 201 OLIN LIBRARY CORNELL UNIVERSITY ITHACA, NY 14853, 17 August 2018 (2018-08-17), XP081257994, DOI: 10.1109/LRA.2018.2858744
- YU HAORAN ET AL: "Calibration and Integration of B-Mode Optical Coherence Tomography for Assistive Control in Robotic Microsurgery", IEEE / ASME TRANSACTIONS ON MECHATRONICS, IEEE SERVICE CENTER, PISCATAWAY, NJ, US, vol. 21, no. 6, December 2016 (2016-12-01), pages 2613 - 2623, XP011633558, ISSN: 1083-4435, [retrieved on 20161109], DOI: 10.1109/TMECH.2016.2583259
- OSCAR M. CARRASCO-ZEVALLOS ET AL: "Review of intraoperative optical coherence tomography: technology and applications [Invited]", BIOMEDICAL OPTICS EXPRESS, vol. 8, no. 3, 21 February 2017 (2017-02-21), United States, pages 1607 - 1637, XP055487817, ISSN: 2156-7085, DOI: 10.1364/BOE.8.001607

## Description

### Domaine technique de l'invention

L'invention a trait aux procédés et aux dispositifs chirurgicaux. En Particulier aux procédés et aux dispositifs chirurgicaux de l'œil.

La chirurgie de l'œil est une intervention délicate. Les outils chirurgicaux utilisés présentent des dimensions (de l'ordre de quelques centaines de micromètres) adaptées à la zone chirurgicale et sont déplacés dans l'œil, sur la zone chirurgicale à traiter dont la surface s'étend de quelques millimètres carrés à un centimètre carré environ.

Certaines interventions nécessitent d'insérer des outils à l'intérieur de l'œil. C'est le cas notamment de la chirurgie de la rétine qui nécessite d'insérer plusieurs outils chirurgicaux à travers le corps vitré pour atteindre la rétine, laquelle tapisse le fond de l'oeil.

Une intervention chirurgicale nécessite l'utilisation d'un dispositif chirurgical qui comporte :
- au moins un outil chirurgical,
- des systèmes d'imagerie,
- un écran pour afficher les images obtenues grâce aux systèmes d'imagerie.

Les systèmes d'imagerie comportent un microscope ophtalmologique d'une part pour visualiser la surface de la rétine et un imageur permettant d'obtenir une image par tomographie optique cohérente située dans le champs de l'image du microscope ophtalmologique, et connue sous l'acronyme anglais OCT ou français TCO, d'autre part. L'image OCT est une vue en profondeur de l'œil. Plus précisément, il s'agit d'une vue en coupe de l'œil selon un ou plusieurs axes de coupe, c'est-à-dire en deux dimensions, qui permet de visualiser les structures anatomiques de la rétine en profondeur. L'image obtenue par OCT est affichée sur l'écran ou dans le microscope ophtalmologique. Le microscope ophtalmologique et l'imageur OCT apportent des informations importantes et complémentaires au chirurgien.

D'autres techniques d'imagerie en profondeur sur une coupe sont connues de l'art antérieur. Les ultrasons, SM-OCT (Speckle-Modulating OCT), SS-OCT (Swept-Source OCT), SD-OCT (Spectral-Domain OCT) ou OCTA (OCT Angiography) sont des techniques d'imagerie permettant d'obtenir une vue en profondeur de l'œil et plus précisément une vue en coupe de l'œil selon un ou plusieurs axes de coupe.

Ces techniques sont notamment décrites par M. Zhou et al., "Towards Robotic Eye Surgery: Marker-Free, Online Hand-Eye Calibration Using optical Coherence Tomography Images" in IEEE Robotics and Automation Letters, vol.3, no. 4, pp. 3944-3951, Oct. 2018, Y. Haoran et al., "Calibration and Integration of B-Mode Optical Coherence Tomography for Assistive Control in Robotic Microsurgery", in IEEE/ASME Transactions on Mechatronics, vol.21, no.6, pp. 2613-2633, Dec. 2016 ou encore O. M . Carrasco-Zevallos et al., "Review of intraoperative optical coherence tomography: technology and applications [Invited]", Biomed. Opt. Express 8, 1607-1637, 2017.

Au cours de l'intervention, l'outil chirurgical est inséré à l'intérieur de l'œil et traverse le corps vitré. L'extrémité de l'outil chirurgical est mise en contact avec la zone chirurgicale. Pendant l'intervention, le chirurgien manœuvre l'outil chirurgical sur la zone chirurgicale. Afin de guider l'extrémité de l'outil chirurgical, le chirurgien a besoin de visualiser les structures anatomiques où l'extrémité de l'outil chirurgical se situe, il déplace donc l'axe de coupe de l'imageur OCT pour que l'image OCT acquise coïncide en permanence avec l'extrémité dudit outil chirurgical. Ceci permet de visualiser sur l'écran, les structures anatomiques à l'extrémité de l'outil chirurgical pendant l'intervention. Le chirurgien doit déplacer l'axe de coupe OCT car le déplacement de l'extrémité de l'outil chirurgical, fait que le champ de l'imageur OCT ne correspond plus à la position de l'extrémité dudit outil chirurgical.

En pratique, le chirurgien déplace l'outil chirurgical avec sa main et déplace l'axe de coupe de l'imageur OCT à l'aide d'une commande actionnable avec le pied ou joystick à pied, telles que des pédales ou joystick à pied. En effet, de manière générale, le chirurgien utilise ses deux mains pour manipuler deux outils chirurgicaux (une pince et un chandelier pour l'éclairage, par exemple) et seuls ses pieds sont disponibles pour déplacer l'axe de coupe OCT et ajuster l'agrandissement et la mise au point du microscope ophtalmologique. Une des difficultés à exécuter la chirurgie est donc liée au maniement simultané de plusieurs instruments chirurgicaux et systèmes d'imagerie. Une autre difficulté rencontrée par les chirurgiens dans ce type d'intervention, est le tremblement naturel des mains ou des mouvements brusques, qui peuvent être dangereux compte tenu des dimensions de la zone chirurgicale.

Un objectif de l'invention est de réduire le nombre de manipulations simultanées du chirurgien.

Un autre objectif est de limiter les conséquences qu'un mouvement non souhaité pourrait avoir sur un patient.

Un autre objectif est de permettre des mouvements et des positionnements de l'outil chirurgical plus précis que ceux réalisés par les techniques connues.

### Résumé de l'invention

A cet effet, l'invention concerne un dispositif chirurgical tel que défini par la revendication 1.

Des caractéristiques supplémentaires font l'objet des revendications dépendantes.

La portée de l'invention est définie par les revendications annexées.

L'unité de commande permet avantageusement de réduire le nombre de manipulations simultanées du chirurgien. Elle permet aussi de limiter les conséquences qu'un mouvement non souhaité pourrait avoir sur un patient et de réaliser des mouvements et des positionnements de l'outil chirurgical plus précis que ceux réalisés par les techniques connues.

### Résumé de la présente description

La présente description concerne un dispositif chirurgical tel que décrit en détail ci-dessous.

Diverses caractéristiques supplémentaires peuvent être prévues seules ou en combinaison :
- l'outil d'imagerie est apte à acquérir une première image en profondeur selon un premier axe de coupe dit horizontal et une deuxième image en profondeur selon un deuxième axe de coupe dit vertical, les axes de coupe se croisant en un point d'intersection, et dans lequel l'unité de commande est apte à envoyer des informations vers l'élément mobile robotisé et/ou l'outil d'imagerie de sorte que la position du point d'intersection coïncide sensiblement avec la position de l'extrémité distale de l'outil chirurgical ;
- le dispositif chirurgical comprend un outil de commande de l'élément mobile robotisé, l'outil de commande étant apte à envoyer des informations à l'unité de commande ;
- l'élément mobile robotisé est un bras mobile robotisé ;
- le point d'intersection est apte à être déplacé, et dans lequel l'unité de commande est apte à envoyer des informations à l'élément mobile robotisé de sorte que l'extrémité distale de l'outil chirurgical coïncide sensiblement avec le point d'intersection en temps réel ;
- le point d'intersection est apte à être déplacé, et dans lequel l'unité de commande est apte à envoyer des informations à l'outil d'imagerie de sorte que le point d'intersection coïncide sensiblement avec l'extrémité distale de l'outil chirurgical en temps réel ;
- le point d'intersection est apte à être déplacé à une position donnée, et dans lequel l'unité de commande est apte à envoyer une information au élément mobile robotisé de sorte que l'extrémité distale de l'outil chirurgical coïncide sensiblement avec le point d'intersection lorsque ledit point d'intersection est positionné à ladite position donnée ;
- l'élément mobile robotisé est apte à être déplacé, et dans lequel l'unité de commande est apte à envoyer des informations à l'élément mobile robotisé de sorte que l'extrémité distale de l'outil chirurgical coïncide sensiblement avec le point d'intersection en temps réel ;
- le dispositif chirurgical comprend un microscope chirurgical apte à acquérir une image microscopique en deux dimensions d'une surface anatomique, ladite image microscopique comportant la zone d'acquisition et les axes de coupe de l'outil d'imagerie en profondeur, ladite image microscopique étant projetée sur l'écran ;
- le dispositif chirurgical est apte à afficher sur l'image OCT, une position de l'outil chirurgical ;
- le dispositif chirurgical est apte à afficher sur l'image microscopique, une position de l'outil chirurgical.

La présente description concerne aussi un procédé de mise en œuvre d'un dispositif chirurgical tel que précédemment décrit, ledit procédé comprenant les étapes suivantes :
- une étape d'envoi d'une consigne de déplacement à l'outil de commande,
- une étape d'acquisition par l'unité de commande de la consigne de déplacement de l'élément mobile robotisé ou de la zone d'acquisition du système d'imagerie,
- une étape dans laquelle une commande est envoyée à l'élément mobile robotisé ou à la zone d'acquisition de sorte à déplacer l'outil chirurgical ou la zone d'acquisition pour que la position de la zone d'acquisition coïncide sensiblement avec la position de l'extrémité de l'outil chirurgical.

Diverses caractéristiques supplémentaires peuvent être prévues seules ou en combinaison :
- le procédé comprend une opération de recalage initiale destinée à étalonner le dispositif chirurgical, ladite opération de recalage permettant à l'unité de commande de calculer une matrice de transformation permettant de transformer tout point dans le système de coordonnés des images en profondeur et de l'image microscopique, en position dans le système de coordonnés de l'élément mobile robotisé et inversement ;
- l'opération de recalage comprend :
- une première étape dans laquelle l'outil chirurgical est déplacé dans une image microscopique, de sorte qu'une extrémité distale dudit outil chirurgical est visible sur ladite image microscopique,
- une deuxième étape dans laquelle la position de l'extrémité distale est identifiée manuellement ou automatiquement sur l'image microscopique,
- une troisième étape dans laquelle la position de la zone d'acquisition est mise à jour de sorte que ladite zone d'acquisition coïncide avec l'extrémité distale de l'outil chirurgical,
- une quatrième étape dans laquelle la coordonnée en profondeur de l'extrémité distale est identifiée manuellement ou automatiquement sur au moins une image OCT, procédé dans lequel, l'opération de recalage est réalisée à plusieurs reprises pour différentes positions de l'extrémité distale, de préférence à trois reprises.

### Brève description des figures

D'autres particularités et avantages de l'invention apparaîtront encore dans la description ci-après en relation avec les dessins annexés, donnés à titre d'exemples non limitatifs :
[Fig.1] la figure 1 est une représentation schématique d'un dispositif chirurgical selon l'invention ;
[Fig.2] la figure 2 est une représentation schématique du dispositif chirurgical en cours d'utilisation ;
[Fig.3] la figure 3 est une représentation schématique d'un œil sur lequel est pratiquée une intervention chirurgicale ;
[Fig.4a] la figure 4a est une image d'une intervention chirurgicale ;
[Fig.4b] la figure 4b est une image d'une intervention chirurgicale ;
[Fig.4c] la figure 4c est une image d'une intervention chirurgicale ;
[Fig.5] la figure 5 est une représentation schématique d'un procédé de mise en œuvre du dispositif chirurgical selon l'invention ;
[Fig.6] la figure 6 est une image réelle d'une intervention chirurgicale dans laquelle la position d'un outil chirurgical est ajoutée par logiciel.

### Description détaillée de l'invention

Dans la description détaillée qui suit, le cas particulier, non limitatif, de l'utilisation d'un imageur permettant d'obtenir une image OCT est décrit.

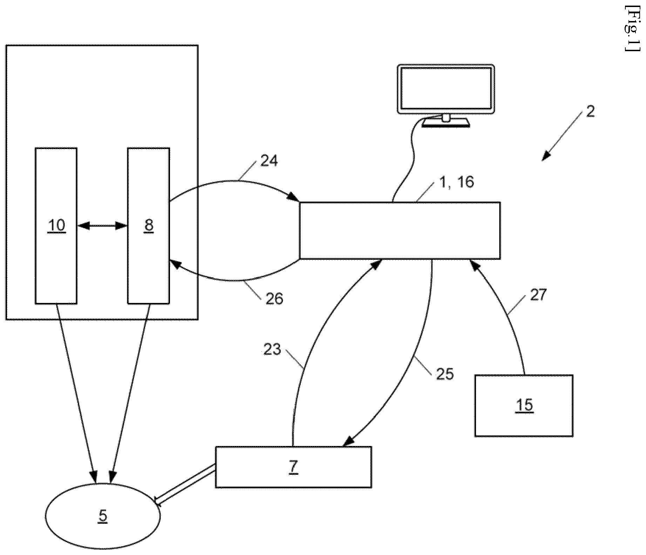

Sur la figure 1, est représentée une unité 1 de commande d'un dispositif 2 chirurgical.

Dans le mode de réalisation représenté sur les figures, le dispositif 2 chirurgical est apte et destiné à effectuer des interventions chirurgicales sur un œil 6.

Le dispositif 2 chirurgical comprend un outil 3 chirurgical comportant une extrémité 4, dite distale, destinée à venir au contact d'un patient 5, et plus précisément apte à être positionnée au moins en profondeur dans l'œil 6 d'un patient 5.

Le dispositif 2 chirurgical comprend au moins un élément mobile 7 robotisé sur lequel est monté l'outil 3 chirurgical. L'élément mobile 7 robotisé est apte à déplacer l'outil 3 chirurgical. Dans le mode de réalisation représenté sur les figures, l'élément mobile 7 est un bras mobile.

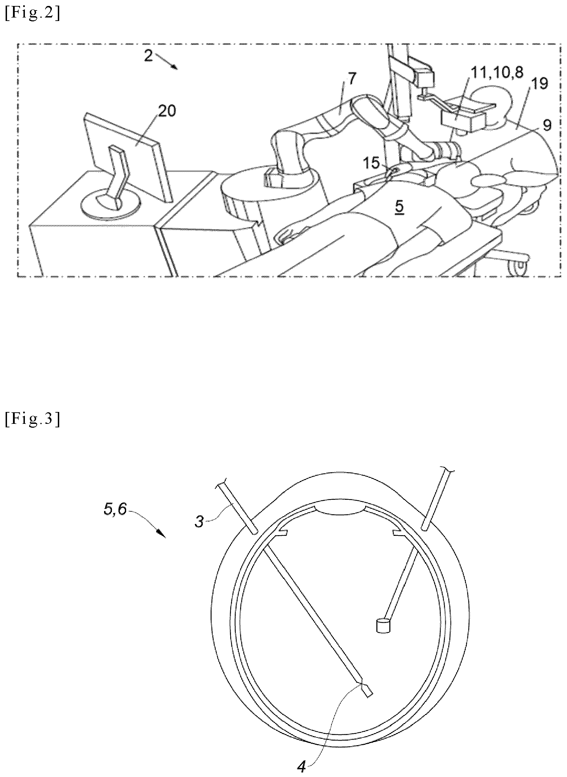

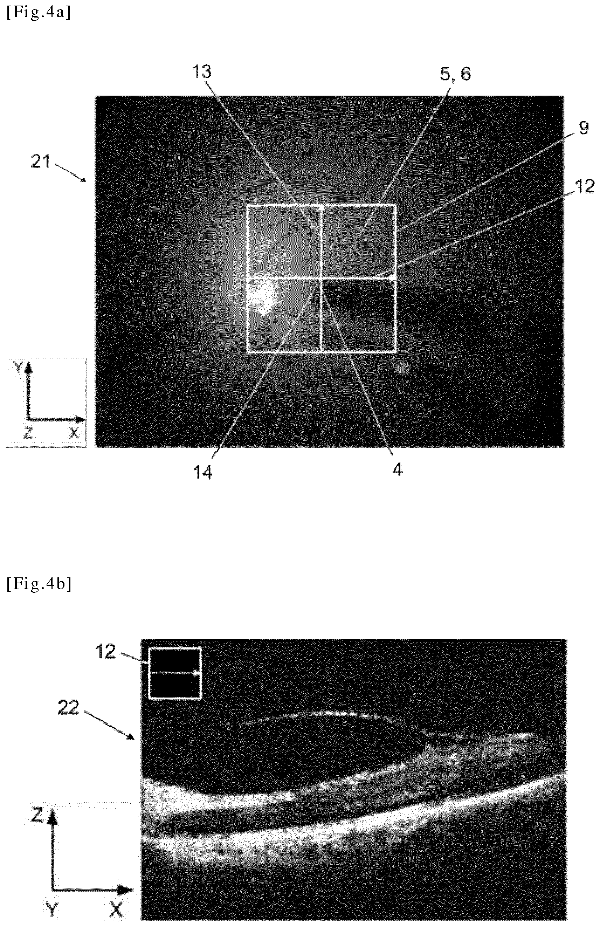

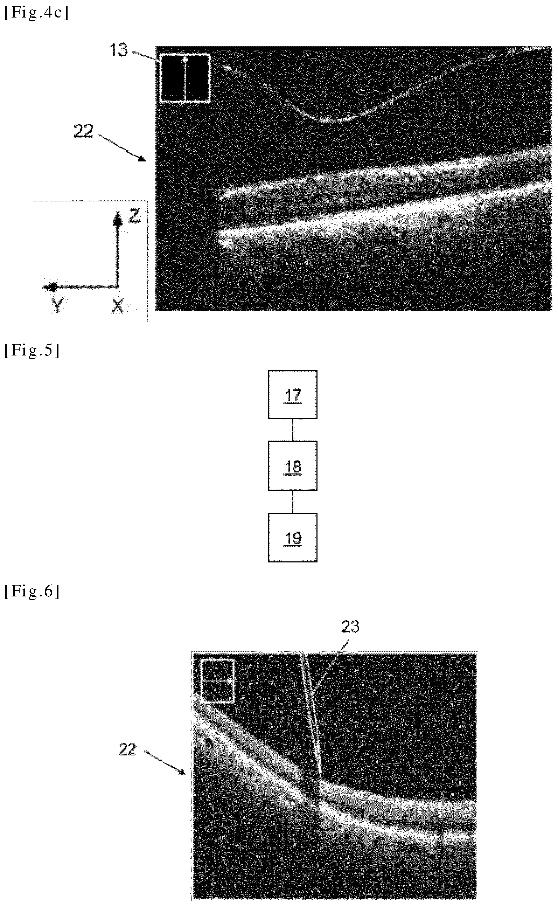

Le dispositif 2 chirurgical comprend un imageur 8 OCT apte à acquérir une image en profondeur au niveau d'une zone 9 d'acquisition sur le patient 5. L'imageur 8 OCT peut déplacer la zone 9 d'acquisition sur le patient 5. Les images obtenues à partir de l'imageur 8 OCT sont appelées dans la suite « images 22 OCT ». Le dispositif 2 chirurgical comprend un microscope 10 chirurgical, apte à acquérir une image d'une surface anatomique telle que la rétine de l'œil 6 par exemple. Dans le mode de réalisation représenté sur les figures, le microscope 10 chirurgical est un microscope ophtalmologique. Le microscope 10 ophtalmologique est colocalisé avec l'imageur 8 OCT dans un seul dispositif 11 d'imagerie visible sur la figure 2. Les images obtenues à partir du microscope 10 ophtalmologique sont des images microscopiques qui seront appelées dans la suite « images 21 ophtalmoscopiques ». La zone 9 d'acquisition se présente sous la forme d'un carré traversé par plusieurs axes 12, 13 de coupe ainsi qu'illustré sur la figure 4a. Sur cette figure, sont visibles un axe de coupe 12 horizontal et un axe de coupe 13 vertical qui se croisent en un point 14 d'intersection. La figure 4b représente les structures anatomiques d'une rétine selon l'axe de coupe 12 horizontal et la figure 4c représente les structures anatomiques de la rétine selon l'axe de coupe 13 vertical.

L'unité 1 de commande est apte à envoyer des informations 25 à l'élément mobile 7 robotisé et/ou des informations 26 à l'imageur 8 OCT de sorte que la position de l'axe de coupe 13 vertical et de l'axe de coupe 12 horizontal coïncident avec la position de l'extrémité 4 distale de l'outil 3 chirurgical. En d'autres termes le point 14 d'intersection coïncide avec l'extrémité 4 distale. A cet effet, l'unité 1 de commande est apte à recevoir des informations 23 sur la position de l'élément mobile 7 robotisé, ces informations 23 de position provenant dudit élément mobile robotisé. L'unité 1 de commande est également apte à recevoir des données 24 des images 22, 21 OCT et ophtalmologique respectivement de l'imageur 8 OCT et du microscope 10 ophtalmologique et envoyer des informations 26 de positionnement à l'imageur OCT.

Le chirurgien 19 n'intervient pas directement sur le patient 5, mais sur un outil 15 de commande visible sur la figure 2. Ainsi le dispositif 2 chirurgical comporte en outre un outil 15 de commande apte à envoyer des informations 27 de déplacement de l'élément mobile robotisé à l'unité 1 de commande. Dans le mode de réalisation représenté sur les figures, l'outil 15 de commande est un stylet de pilotage.

Avant de débuter chaque intervention chirurgicale, une opération de recalage initiale est réalisée en dehors du patient. L'opération de recalage consiste à étalonner le dispositif 2 chirurgical de sorte que l'unité 1 de commande connaisse la position de l'outil 3 chirurgical par rapport aux axes de coupe 12, 13.

A cet effet, l'unité 1 de commande comporte une unité 16 de stockage dans laquelle est stocké une matrice de transformation. La matrice de transformation permet de synchroniser les positions des axes de coupes 12, 13 et la profondeur dans les images 22 OCT avec la position de l'élément mobile 7 robotisé.

Deux opérations de recalage initiales peuvent être utilisées. La première opération de recalage est réalisée en suivant des étapes successives, décrites ci-après.

Dans une première étape l'outil 3 chirurgical porté par l'élément mobile 7 robotisé est déplacé dans le champs du microscope 10 ophtalmologique, c'est-à-dire de sorte que l'extrémité 4 distale soit visible dans l'image 21 ophtalmoscopique, ceci étant effectué à l'aide de l'outil 15 de commande. Dans une deuxième étape, le chirurgien 19 identifie la position de l'extrémité 4 distale sur l'image 21 ophtalmoscopique. Cette étape peut être réalisée de manière automatique ou manuelle. De manière automatique, l'unité 1 de commande peut détecter l'extrémité 4 distale de l'outil 3 chirurgical sur l'image 21 ophtalmoscopique et déterminer les coordonnés selon les deux dimensions (x, y) de ladite extrémité 4 distale. En mode manuel, l'extrémité 4 distale est visible sur l'écran 20. Le chirurgien 19, sélectionne l'extrémité 4 distale avec une souris, pavé tactile, joystick ou tout autre moyen d'interaction homme machine (IHM). La sélection est effectuée sur l'image 21 ophtalmoscopique de la figure 4a pour obtenir les coordonnés (x, y). Dans une troisième étape, l'unité 1 de commande met à jour la position de la zone 9 d'acquisition et des axes de coupe 12, 13 de sorte que les deux axes de coupe 12, 13 coïncident avec l'extrémité 4 distale de l'outil 3 chirurgical. Ainsi, l'extrémité 4 distale de l'outil 3 chirurgical se situe à une intersection des deux axes de coupe 12, 13. Dans une quatrième étape le chirurgien sélectionne l'extrémité 4 distale sur un des deux axes de coupe pour obtenir la coordonnée en z. Cette étape peut être réalisée de manière automatique ou manuelle. De manière automatique, l'unité 1 de commande peut détecter l'extrémité 4 distale de l'outil 3 chirurgical sur l'image OCT et ainsi déterminer la coordonnée en z.

Pour des cas où l'image 21 ophtalmoscopique et les deux axes de coupe 12, 13 n'ont pas de distorsion, un point peut être suffisant. Dans ce cas, le recalage entre le robot et l'image est basé sur un algorithme de recalage rigide. Si, par contre, une distorsion existe dans l'image, plusieurs points doivent être acquis, de préférence avec un point dans le centre de la zone 9 d'acquisition et plusieurs points dans les parties périphériques de la zone 9 d'acquisition. Le recalage entre le robot et l'image est dans ce cas basé sur un algorithme de recalage non-rigide. Plus il y a de points, mieux sera la précision. Pour guider l'utilisateur, l'unité 1 de commande peut afficher une valeur erreur RMS (Root Mean Squared) à la suite de chaque point ajouté qui donne une indication de la précision avec ce nombre de points.

Une fois le recalage achevé, l'unité 1 de commande calcule une matrice de transformation à l'aide d'un algorithme de recalage standard. La matrice de transformation permet de transformer tout point dans le système de coordonnés de l'image OCT et de l'image ophtalmoscopique en position dans le système de coordonnés de l'élément mobile robotisé et inversement.

Selon le choix du chirurgien, une seconde opération de recalage différente de la première opération de recalage, peut être utilisée. Dans une première étape l'instrument chirurgical porté par le robot est déplacé dans la zone d'acquisition de l'imageur OCT, laquelle se trouve dans le champ du microscope ophtalmologique à l'instar de la première procédure de recalage. Dans une deuxième étape, l'axe de coupe horizontal est déplacé en direction de l'extrémité de l'instrument chirurgical jusqu'à apercevoir ladite extrémité sur l'image OCT affichée sur l'écran. Le chirurgien indique à l'unité de commande la position de l'extrémité de l'outil chirurgical lorsqu'il aperçoit celle-ci sur l'image OCT. Cette opération est aussi effectuée avec l'axe de coupe vertical dans un second temps. Comme pour la première procédure, cette opération doit être répétée plusieurs fois pour obtenir une erreur RMS suffisamment petite pour l'application ciblée.

Une fois l'opération de recalage achevée, le chirurgien peut diriger l'élément 7 mobile robotisé à l'aide de l'outil de commande tandis que l'imageur OCT déplace la zone d'acquisition et les axes de coupes horizontal et vertical de sorte que le point d'intersection coïncide avec la position de l'extrémité distale de l'outil chirurgical. Inversement, le chirurgien peut sélectionner un point sur l'imagerie, et l'unité de commande ordonne à l'élément mobile robotisé de se déplacer pour faire en sorte que l'extrémité distale de l'outil chirurgical coïncide avec ce point.

Lorsque l'opération de recalage est achevée, le procédé de mise en œuvre du dispositif chirurgical comprend une étape 17 d'envoi d'une consigne de déplacement à l'aide de l'outil 15 de commande. Le procédé comprend ensuite une étape 18 d'acquisition d'une consigne de déplacement de l'élément mobile robotisé ou de la zone d'acquisition de l'outil d'imagerie et une étape 19 dans laquelle une commande est envoyée à l'élément mobile robotisé ou à la zone d'acquisition de sorte à déplacer l'outil chirurgical ou la zone d'acquisition pour que la position de la zone d'acquisition coïncide sensiblement avec la position de l'extrémité de l'outil chirurgical. Une commande est un ensemble d'instructions ou une seule instruction. Le procédé de mise en œuvre du dispositif chirurgical peut être réalisé en dehors du patient. Autrement dit, le procédé de mise en œuvre peut être réalisé lorsque aucun élément du dispositif chirurgical n'est au contact du patient.

Avantageusement, le dispositif est apte à afficher une position 23 de l'instrument sur l'image 22 OCT. En référence à la figure 6, on peut voir que l'outil 3 chirurgical est affiché superposé sur l'image 22 OCT. Ceci permet avantageusement au praticien de vérifier la précision du recalage. En effet, il peut alors s'assurer que l'outil 3 chirurgical coïncide bien avec la position 23 dessinée sur l'image 22 OCT.

Plusieurs utilisations différentes du dispositif chirurgical sont possibles. Ces utilisations sont décrites ci-après.

### Première option : Asservissement de la position de l'imageur OCT

Selon une première utilisation possible du dispositif chirurgical, le chirurgien déplace l'élément mobile robotisé à l'aide de l'outil de commande. L'unité de commande ordonne à l'imageur OCT de déplacer la zone d'acquisition et les axes de coupe vertical et horizontal de sorte que le point d'intersection coïncide avec l'extrémité distale de l'outil chirurgical.

Ainsi, le chirurgien peut visualiser sur l'écran, les structures anatomiques et l'extrémité distale de l'outil chirurgical en temps réel.

### Deuxième option : Asservissement de la position de l'outil chirurgical

Selon une deuxième utilisation possible du dispositif chirurgical, le chirurgien déplace la zone d'acquisition et par conséquent les axes de coupe horizontal et vertical. Ainsi le chirurgien peut visualiser un endroit anatomique en profondeur puis décider d'intervenir. Lorsqu'il décide d'intervenir, il indique à l'unité de commande de déplacer l'extrémité distale du dispositif chirurgical au point de coordonnés correspondant à l'intersection des axes de coupe horizontal et vertical (x, y) et sur un point en profondeur indiqué sur l'image OCT (z). En variante, l'élément mobile robotisé se déplace instantanément en suivant en temps réel un point défini sur les images microscopiques et OCT. Ce point peut être un repère anatomique identifié par le chirurgien et qui se déplace suite aux mouvements du patient (respiration, battement du cœur). Le mouvement de ce repère est calculé par l'unité de commande à base de l'image microscopique et/ou les images OCT en utilisant un algorithme de suivi.

Il devient alors possible d'effectuer des chirurgies de haute précision grâce à l'élément mobile robotisé qui se déplace à un endroit anatomique sélectionné par le chirurgien. Cette utilisation est particulièrement utile lorsque l'extrémité distale de l'outil chirurgical doit être placée au centre d'un vaisseau rétinien dont le diamètre peut être inférieur à 10 µm ou encore lorsque ladite extrémité distale doit être positionnée à une profondeur précise sous la surface de la rétine pour effectuer une injection subrétinienne par exemple. En effet, ce type d'intervention est extrêmement difficile car aucun mouvement brusque ou tremblement n'est permis. En effet, le tremblement naturel d'un chirurgien est de l'ordre de 100 µm alors que l'intervention est réalisée sur quelques micromètres.

### Troisième option : Définition de zones interdites sur l'image OCT

Selon une troisième utilisation possible du dispositif chirurgical, il est possible de définir des zones interdites où l'extrémité distale de l'outil chirurgical ne peut se rendre.

Sur les images OCT et sur l'image ophtalmoscopique, le chirurgien définit sur l'écran des zones interdites de sorte que l'unité de commande interdise l'accès à l'extrémité distale auxdites zones interdites. Ceci permet de sécuriser le patient en protégeant des zones anatomiques déterminées contre un mouvement involontaire brusque ou des tremblements du chirurgien.

### Quatrième option : Définition d'un balayage automatique

Selon une quatrième utilisation possible du dispositif chirurgical, il est possible de définir un trajet en trois et/ou deux dimensions sur l'écran à l'aide des images OCT et/ou de l'image ophtalmoscopique. Une fois le trajet dessiné et validé, l'unité de commande ordonne au dispositif chirurgical de suivre le trajet. Ceci permet de réaliser des interventions chirurgicales en suivant des trajets complexes avec un mouvement précis et maitrisé, par exemple pour le traitement par laser de la rétine. Dans cette utilisation, le dispositif chirurgical est par exemple un émetteur laser intraoculaire.

L'utilisation d'une technique d'imagerie par OCT décrite dans le cas particulier qui précède n'est pas limitative pour l'invention. Tout type d'imagerie permettant d'obtenir une image en profondeur sur une coupe peut être utilisée. Cela concerne par exemple, les techniques par ultrasons, SM-OCT (Speckle-Modulating OCT), SS-OCT (Swept-Source OCT), SD-OCT (Spectral-Domain OCT) ou OCTA (OCT Angiography).

La portée de l'invention est définie par les revendications annexées.

## Revendications

1. Dispositif (2) chirurgical comprenant :
- au moins un outil (3) chirurgical comportant une extrémité (4), dite distale, configurée pour venir au contact d'un patient (5), et configurée pour être positionnée dans le patient (5),
- au moins un élément mobile (7) robotisé sur lequel est monté l'outil (3) chirurgical, l'élément mobile (7) robotisé étant configuré pour déplacer ledit outil (3) chirurgical,
- au moins un outil (8) d'imagerie en profondeur configuré pour acquérir au moins une image (22) en profondeur au niveau d'une zone (9) d'acquisition sur le patient (5), ledit outil (8) d'imagerie étant configuré pour déplacer la zone (9) d'acquisition sur le patient (5), ledit au moins un outil (8) d'imagerie étant indépendant dudit au moins un élément mobile (7) robotisé,
- un outil (15) de commande configuré pour ordonner un déplacement indépendant de l'au moins un élément mobile (7) robotisé et de l'outil (8) d'imagerie,
- une unité (1) de commande configurée pour recevoir des informations de l'outil (15) de commande et pour ordonner le déplacement de l'au moins un élément mobile (7) robotisé et/ou l'outil (8) d'imagerie de sorte que la position de la zone (9) d'acquisition coïncide sensiblement avec la position de l'extrémité (4) distale de l'outil (3) chirurgical, ladite unité de commande étant configurée pour recevoir des informations de position venant dudit au moins un élément mobile (7) robotisé et des informations venant de l'outil (8) d'imagerie.

2. Dispositif (2) chirurgical selon la revendication 1 dans lequel l'outil (8) d'imagerie est configuré pour acquérir une première image en profondeur selon un premier axe de coupe (12) dit horizontal et une deuxième image en profondeur selon un deuxième axe de coupe (13) dit vertical, les axes de coupe (12, 13) se croisant en un point (14) d'intersection, et dans lequel l'unité (1) de commande est configurée pour envoyer des informations vers l'élément mobile (7) robotisé et/ou l'outil (8) d'imagerie de sorte que la position du point (14) d'intersection coïncide sensiblement avec la position de l'extrémité (4) distale de l'outil (3) chirurgical.

3. Dispositif (2) chirurgical selon la revendication 2 dans lequel l'élément mobile (7) robotisé est un bras mobile robotisé.

4. Dispositif (2) chirurgical selon l'une des revendications 2 ou 3 dans lequel le point (14) d'intersection est configuré pour être déplacé, et dans lequel l'unité (1) de commande est configuré pour envoyer des informations à l'élément mobile (7) robotisé de sorte que l'extrémité (4) distale de l'outil (3) chirurgical coïncide sensiblement avec le point (14) d'intersection en temps réel.

5. Dispositif (2) chirurgical selon l'une des revendications 3 ou 4 dans lequel le point (14) d'intersection est configuré pour être déplacé, et dans lequel l'unité (1) de commande est configurée pour envoyer des informations à l'outil (8) d'imagerie de sorte que le point (14) d'intersection coïncide sensiblement avec l'extrémité (4) distale de l'outil (3) chirurgical en temps réel.

6. Dispositif (2) chirurgical selon l'une des revendications 3 à 5 dans lequel le point (14) d'intersection est configuré pour être déplacé à une position donnée, et dans lequel l'unité (1) de commande est configurée pour envoyer une information à l'élément mobile (7) robotisé de sorte que l'extrémité (4) distale de l'outil (3) chirurgical coïncide sensiblement avec le point (14) d'intersection lorsque ledit point (14) d'intersection est positionné à ladite position donnée.

7. Dispositif (2) chirurgical selon l'une des revendications 3 à 6 dans lequel l'élément mobile (7) robotisé est configuré pour être déplacé, et dans lequel l'unité (1) de commande est configurée pour envoyer des informations à l'élément mobile (7) robotisé de sorte que l'extrémité (4) distale de l'outil (3) chirurgical coïncide sensiblement avec le point (14) d'intersection en temps réel.

8. Dispositif (2) chirurgical selon l'une des revendications 3 à 7 dans lequel celui-ci comprend un microscope (10) chirurgical configuré pour acquérir une image (21) microscopique en deux dimensions d'une surface anatomique, ladite image (21) microscopique comportant la zone (9) d'acquisition et les axes de coupe (12, 13) de l'outil (8) d'imagerie en profondeur, ladite image (21) microscopique étant projetée sur un écran (20).

9. Dispositif (2) chirurgical selon l'une des revendications précédentes dans lequel celui-ci est configuré pour afficher sur une image (22) OCT, une position (23) de l'outil (3) chirurgical.

10. Dispositif (2) chirurgical selon l'une des revendications 1 à 8 dans lequel celui-ci est configuré pour afficher une image (22) en profondeur obtenue par ultrasons, SM-OCT, SS-OCT, SD-OCT ou OCTA.

11. Dispositif (2) chirurgical selon la revendication 9 ou 10 dans lequel celui-ci est configuré pour afficher sur une image (21) microscopique, une position (23) de l'outil (3) chirurgical.

12. Procédé de mise en œuvre d'un dispositif (2) chirurgical selon la revendication 11, ledit procédé, réalisé en dehors du patient, comprenant les étapes suivantes :
- une étape d'envoi (17) d'une consigne de déplacement à l'outil (15) de commande,
- une étape d'acquisition (18) par l'unité (1) de commande de la consigne de déplacement de l'élément mobile (7) robotisé ou de la zone (9) d'acquisition de l'outil (8) d'imagerie,
- une étape dans laquelle l'unité (1) de commande reçoit des informations de position venant dudit élément mobile (7) robotisé ou des informations venant de l'outil (8) d'imagerie,
- une étape (19) dans laquelle une commande est envoyée à l'élément mobile (7) robotisé ou à la zone (9) d'acquisition de sorte à déplacer l'outil (3) chirurgical ou la zone (9) d'acquisition pour que la position de la zone (9) d'acquisition coïncide sensiblement avec la position de l'extrémité de l'outil (3) chirurgical.

13. Procédé de mise en œuvre d'un dispositif (2) chirurgical selon la revendication 12 dans lequel celui-ci comprend une opération de recalage initiale destinée à étalonner le dispositif (2) chirurgical, ladite opération de recalage permettant à l'unité (1) de commande de calculer une matrice de transformation permettant de transformer tout point dans le système de coordonnés des images en profondeur et de l'image (21) microscopique, en position dans le système de coordonnés de l'élément mobile (7) robotisé et inversement.

14. Procédé de mise en œuvre d'un dispositif (2) chirurgical selon la revendication 13 dans lequel l'opération de recalage comprend :
- une première étape dans laquelle l'outil (3) chirurgical est déplacé dans une image (21) microscopique, de sorte qu'une extrémité (4) distale dudit outil (3) chirurgical est visible sur ladite image (21) microscopique,
- une deuxième étape dans laquelle la position de l'extrémité (4) distale est identifiée manuellement ou automatiquement sur l'image (21) microscopique,
- une troisième étape dans laquelle la position de la zone (9) d'acquisition est mise à jour de sorte que ladite zone (9) d'acquisition coïncide avec l'extrémité (4) distale de l'outil (3) chirurgical,
- une quatrième étape dans laquelle la coordonnée en profondeur de l'extrémité (4) distale est identifiée manuellement ou automatiquement sur au moins une image (22) OCT,
procédé dans lequel, l'opération de recalage est réalisée à plusieurs reprises pour différentes positions de l'extrémité (4) distale, de préférence à trois reprises.

## Patentansprüche

1. Chirurgische Vorrichtung (2), umfassend:
- mindestens ein chirurgisches Werkzeug (3), das ein als distal bezeichnetes Ende (4) umfasst, das dazu ausgelegt ist, einen Patienten (5) zu berühren, und dazu ausgelegt ist, im Patienten (5) positioniert zu werden,
- mindestens ein robotisiertes mobiles Element (7), an dem das chirurgische Werkzeug (3) angebracht ist, wobei das robotisierte mobile Element (7) dazu ausgelegt ist, das chirurgische Werkzeug (3) zu verschieben,
- mindestens ein Tiefenbildgebungswerkzeug (8), das dazu ausgelegt ist, mindestens ein Tiefenbild (22) an einem Erfassungsbereich (9) auf dem Patienten (5) zu erfassen, wobei das Bildgebungswerkzeug (8) dazu ausgelegt ist, den Erfassungsbereich (9) auf dem Patienten (5) zu verschieben, wobei das mindestens eine Bildgebungswerkzeug (8) unabhängig von dem mindestens einen robotisierten mobilen Element (7) ist,
- ein Steuerungswerkzeug (15), das dazu ausgelegt ist, eine Verschiebung unabhängig von dem mindestens einen robotisierten mobilen Element (7) und dem Bildgebungswerkzeug (8) zu befehlen,
- eine Steuereinheit (1), die dazu ausgelegt ist, Informationen von dem Steuerungswerkzeug (15) zu empfangen und die Verschiebung des mindestens einen robotisierten mobilen Elements (7) und/oder des Bildgebungswerkzeugs (8) derart zu befehlen, dass die Position des Erfassungsbereichs (9) im Wesentlichen mit der Position des distalen Endes (4) des chirurgischen Werkzeugs (3) zusammenfällt, wobei die Steuereinheit dazu ausgelegt ist, Positionsinformationen, die von dem mindestens einen robotisierten mobilen Element (7) stammen, und Informationen, die von dem Bildgebungswerkzeug (8) stammen, zu empfangen.

2. Chirurgische Vorrichtung (2) nach Anspruch 1, wobei das Bildgebungswerkzeug (8) dazu ausgelegt ist, ein erstes Tiefenbild entlang einer ersten, als horizontal bezeichneten Schnittachse (12) und ein zweites Tiefenbild entlang einer zweiten, als vertikal bezeichneten Schnittachse (13) zu erfassen, wobei sich die Schnittachsen (12, 13) in einem Schnittpunkt (14) schneiden, und wobei die Steuereinheit (1) dazu ausgelegt ist, derart Informationen zu dem robotisierten mobilen Element (7) und/oder dem Bildgebungswerkzeug (8) zu senden, dass die Position des Schnittpunkts (14) im Wesentlichen mit der Position des distalen Endes (4) des chirurgischen Werkzeugs (3) zusammenfällt.

3. Chirurgische Vorrichtung (2) nach Anspruch 2, wobei das robotisierte mobile Element (7) ein robotisierter mobiler Arm ist.

4. Chirurgische Vorrichtung (2) nach einem der Ansprüche 2 oder 3, wobei der Schnittpunkt (14) dazu ausgelegt ist, verschoben zu werden, und wobei die Steuereinheit (1) dazu ausgelegt ist, derart Informationen an das robotisierte mobile Element (7) zu senden, dass das distale Ende (4) des chirurgischen Werkzeugs (3) im Wesentlichen mit dem Schnittpunkt (14) in Echtzeit zusammenfällt.

5. Chirurgische Vorrichtung (2) nach einem der Ansprüche 3 oder 4, wobei der Schnittpunkt (14) dazu ausgelegt ist, verschoben zu werden, und wobei die Steuereinheit (1) dazu ausgelegt ist, derart Informationen an das Bildgebungswerkzeug (8) zu senden, dass der Schnittpunkt (14) im Wesentlichen mit dem distalen Ende (4) des chirurgischen Werkzeugs (3) in Echtzeit zusammenfällt.

6. Chirurgische Vorrichtung (2) nach einem der Ansprüche 3 bis 5, wobei der Schnittpunkt (14) dazu ausgelegt ist, zu einer gegebenen Position verschoben zu werden, und wobei die Steuereinheit (1) dazu ausgelegt ist, derart eine Information an das robotisierte mobile Element (7) zu senden, dass das distale Ende (4) des chirurgischen Werkzeugs (3) im Wesentlichen mit dem Schnittpunkt (14) zusammenfällt, wenn der Schnittpunkt (14) in der gegebenen Position positioniert ist.

7. Chirurgische Vorrichtung (2) nach einem der Ansprüche 3 bis 6, wobei das robotisierte mobile Element (7) dazu ausgelegt ist, verschoben zu werden, und wobei die Steuereinheit (1) dazu ausgelegt ist, derart Informationen an das robotisierte mobile Element (7) zu senden, dass das distale Ende (4) des chirurgischen Werkzeugs (3) im Wesentlichen mit dem Schnittpunkt (14) in Echtzeit zusammenfällt.

8. Chirurgische Vorrichtung (2) nach einem der Ansprüche 3 bis 7, wobei diese ein chirurgisches Mikroskop (10) umfasst, das dazu ausgelegt ist, ein zweidimensionales mikroskopisches Bild (21) einer anatomischen Oberfläche zu erfassen, wobei das mikroskopische Bild einen Erfassungsbereich (9) und die Schnittachsen (12, 13) des Tiefenbildgebungswerkzeugs (8) umfasst, wobei das mikroskopische Bild auf einen Bildschirm (20) projiziert wird.

9. Chirurgische Vorrichtung (2) nach einem der vorstehenden Ansprüche, wobei diese dazu ausgelegt ist, eine Position des chirurgischen Werkzeugs (3) auf einem OCT-Bild (22) anzuzeigen.

10. Chirurgische Vorrichtung (2) nach einem der Ansprüche 1 bis 8, wobei diese dazu ausgelegt ist, ein durch Ultraschall, SM-OCT, SS-OCT, SD-OCT oder OCTA erhaltenes Tiefenbild (22) anzuzeigen.

11. Chirurgische Vorrichtung (2) nach einem der Ansprüche 9 oder 10, wobei diese dazu ausgelegt ist, eine Position (23) des chirurgischen Werkzeugs (3) auf einem mikroskopischen Bild (21) anzuzeigen.

12. Verfahren zum Implementieren einer chirurgischen Vorrichtung (2) nach Anspruch 11, wobei das Verfahren, das außerhalb des Patienten durchgeführt wird, die folgenden Schritte umfasst:
- einen Schritt des Sendens (17) einer Anweisung zur Verschiebung an das Steuerungswerkzeug (15),
- einen Schritt des Erfassens (18), durch die Steuereinheit (1), der Anweisung zur Verschiebung des robotisierten mobilen Elements (7) oder des Erfassungsbereichs (9) des Bildgebungswerkzeugs (8),
- einen Schritt, bei dem die Steuereinheit (1) Positionsinformationen, die von dem robotisierten mobilen Elements (7) stammen, oder Informationen, die von dem Bildgebungswerkzeug (8) stammen, empfängt,
- einen Schritt (19), bei dem ein Befehl derart an das robotisierte mobile Element (7) oder an den Erfassungsbereich (9) gesendet wird, dass das chirurgische Werkzeug (3) oder der Erfassungsbereich (9) verschoben wird, damit die Position des Erfassungsbereichs (9) im Wesentlichen mit der Position des Endes des chirurgischen Werkzeugs (3) zusammenfällt.

13. Verfahren zum Implementieren einer chirurgischen Vorrichtung (2) nach Anspruch 12, wobei dieses einen anfänglichen Neueinstellungsvorgang umfasst, der dazu vorgesehen ist, die chirurgische Vorrichtung (2) zu kalibrieren, wobei der Neueinstellungsvorgang ermöglicht, dass die Steuereinheit (1) eine Transformationsmatrix berechnet, die ermöglicht, jeden Punkt im Koordinatensystem der Tiefenbilder und des mikroskopischen Bilds (21) in eine Position im Koordinatensystem des robotisierten mobilen Systems (7) und umgekehrt umzuwandeln.

14. Verfahren zum Implementieren einer chirurgischen Vorrichtung (2) nach Anspruch 13, wobei der Neueinstellungsvorgang Folgendes umfasst:
- einen ersten Schritt, bei dem das chirurgische Werkzeug (3) derart in einem mikroskopischen Bild (21) verschoben wird, dass ein distales Ende (4) des chirurgischen Werkzeugs (3) auf dem mikroskopischen Bild (21) sichtbar ist,
- einen zweiten Schritt, bei dem die Position des distalen Endes (4) manuell oder automatisch auf dem mikroskopischen Bild (21) identifiziert wird,
- einen dritten Schritt, bei dem die Position des Erfassungsbereichs (9) derart aktualisiert wird, dass der Erfassungsbereich (9) mit dem distalen Ende (4) des chirurgischen Werkzeugs (3) zusammenfällt,
- einen vierten Schritt, bei dem die Tiefenkoordinate des distalen Endes (4) manuell oder automatisch auf mindestens einem OCT-Bild (22) identifiziert wird,
wobei bei dem Verfahren der Neueinstellungsvorgang mehrmals, vorzugsweise dreimal, für unterschiedliche Positionen des distalen Endes (4) durchgeführt wird.

## Claims

1. A surgical device (2) comprising:
- at least one surgical tool (3) comprising an end (4), called distal, configured to come in contact with a patient (5), and configured to be positioned in the patient (5),
- at least one robotic mobile element (7) on which the surgical tool (3) is mounted, the robotic mobile element (7) being configured to move said surgical tool (3),
- at least one depth imaging tool (8) configured to acquire at least one depth image (22) at an acquisition area (9) on the patient (5), said imaging tool (8) being configured to move the acquisition area (9) on the patient (5), said at least one imaging tool (8) being independent of said at least one robotic mobile element (7),
- a control tool (15) configured to order an independent move of said at least one robotic mobile element (7) and said imaging tool (8),
- a control unit (1) configured to receive information from the control tool (15) and to order the move of said at least one robotic mobile element (7) and/or the imaging tool (8) so that the position of the acquisition area (9) substantially coincides with the position of the distal end (4) of the surgical tool (3), said control unit being configured to receive positional information coming from said at least one robotic mobile element (7) and information coming from said imaging tool (8).

2. The surgical device (2) according to claim 1 in which the imaging tool (8) is configured to acquire a first depth image according to a first cutting axis (12), called horizontal, and a second depth image according to a second cutting axis (13), called vertical, the cutting axes (12, 13) intersecting at an intersection point (14), and wherein the control unit (1) is configured to send information to the robotic mobile element (7) and/or the imaging tool (8) so that the position of the intersection point (14) substantially coincides with the position of the distal end (4) of the surgical tool (3).

3. The surgical device (2) according to claim 2 wherein the robotic mobile element (7) is a robotic mobile arm.

4. The surgical device (2) according to claims 2 or 3, wherein the intersection point (14) is configured to be moved, and wherein the control unit (1) is configured to send information to the robotic mobile element (7) such that the distal end (4) of the surgical tool (3) substantially coincides with the intersection point (14) in real time.

5. The surgical device (2) according to any of claims 3 or 4, wherein the intersection point (14) is configured to be moved, and wherein the control unit (1) is configured to send information to the imaging tool (8) so that the intersection point (14) substantially coincides with the distal end (4) of the surgical tool (3) in real time.

6. The surgical device (2) according to any of claims 3 to 5, wherein the intersection point (14) is configured to be moved to a given position, and wherein the control unit (1) is configured to send an information to the robotic mobile element (7) so that the distal end (4) of the surgical tool (3) substantially coincides with the intersection point (14) when said intersection point (14) is positioned at said given position.

7. The surgical device (2) according to any of claims 3 to 6, wherein the robotic mobile element (7) is configured to be moved, and wherein the control unit (1) is configured to send information to the robotic mobile element (7) such that the distal end (4) of the surgical tool (3) substantially coincides with the intersection point (14) in real time.

8. The surgical device (2) according to any of claims 3 to 7, wherein it comprises a surgical microscope (10) configured to acquire a two-dimensional microscopic image (21) of an anatomical surface, said microscopic image (21) comprising the acquisition area (9) and the cutting axes (12, 13) of the depth imaging tool (8), said microscopic image (21) being projected onto a screen (20).

9. The surgical device (2) according to any of the preceding claims, wherein it is configured to display on an OCT image (22), a position (23) of the surgical tool (3).

10. The surgical device (2) according to any of claims 1 to 8, wherein it is configured to display a depth image (22) obtained by ultrasound, SM-OCT, SS-OCT, SD-OCT or OCTA.

11. The surgical device (2) according to claim 9 or claim 10, wherein it is configured to display on a microscopic image (21), a position (23) of the surgical tool (3).

12. A method of operating a surgical device (2) according to claim 11, said method, performed outside the patient, comprising the following steps:
- a step of sending (17) a displacement instruction to the control tool (15),
- a step of acquiring (18), by the control unit (1), the displacement instruction of the robotic mobile element (7) or of the acquisition area (9) of the imaging tool (8),
- a step in which the control unit (1) receives positional information coming from said robotic mobile element (7) or information coming from said imaging tool (8),
- a step (19) in which a command is sent to the robotic mobile element (7) or to the acquisition area (9) so as to move the surgical tool (3) or the acquisition area (9) so that the position of the acquisition area (9) substantially coincides with the position of the end of the surgical tool (3).

13. The method of operating a surgical device (2) according to claim 12, wherein it comprises an initial registration operation for calibrating the surgical device (2), said registration operation allowing the control unit (1) to calculate a transformation matrix allowing to transform any point in the coordinate system of the depth images and the microscopic image (21) into a position in the coordinate system of the robotic mobile element (7) and vice versa.

14. The method of operating a surgical device (2) according to claim 13 wherein the registration operation comprises:
- a first step in which the surgical tool (3) is moved in a microscopic image (21), so that a distal end (4) of said surgical tool (3) is visible in said microscopic image (21),
- a second step in which the position of the distal end (4) is identified manually or automatically in the microscopic image (21),
- a third step in which the position of the acquisition area (9) is updated so that said acquisition area (9) coincides with the distal end (4) of the surgical tool (3),
- a fourth step in which the depth coordinate of the distal end (4) is identified manually or automatically on at least one OCT image (22),
method in which the registration operation is performed several times for different positions of the distal end (4), preferably three times.
